Europäisches Patentamt

European Patent Office

Office européen des brevets

(11) Publication number: **0 393 275 B1**

# EUROPEAN PATENT SPECIFICATION

(45) Date of publication of patent specification: **05.01.94**   (51) Int. Cl.5: **C01F 7/48**, C01F 7/56, C07F 7/00, A61K 7/38

(21) Application number: **89304019.6**

(22) Date of filing: **21.04.89**

(54) **Method for preparing basic aluminum halides.**

(43) Date of publication of application:
**24.10.90 Bulletin 90/43**

(45) Publication of the grant of the patent:
**05.01.94 Bulletin 94/01**

(84) Designated Contracting States:
**DE FR GB IT**

(56) References cited:
**EP-A- 0 047 650**
**EP-A- 0 274 252**
**GB-A- 2 113 666**
**US-A- 4 053 370**

**CHEMICAL ABSTRACTS, vol. 95, no. 20, 1981, page 159, abstract no. 171960d, Columbus, Ohio, US;**

(73) Proprietor: **WESTWOOD CHEMICAL CORPO-RATION**
**PO Box 985**
**Tower Drive**
**Middletown New York 10940/0985(US)**

(72) Inventor: **Givanniello, Rocco**
**9 Painted Apron Terrace**
**Port Jervis New York 12271(US)**
Inventor: **Howe, Stephen M.**
**22 East Avenue**
**Walden New York 12586(US)**

(74) Representative: **Geldard, David Guthrie et al**
**UROUHART-DYKES AND LORD**
**Tower House**
**Merrion Way**
**Leeds West Yorkshire LS2 8PA (GB)**

EP 0 393 275 B1

## Description

### FIELD OF THE INVENTION

This invention relates to basic aluminum halides. In particular, it relates to a process for preparing basic aluminum halide compositions having high antiperspirant activity. Additionally, this invention relates to a process for preparing an improved aluminum zirconium glycinate utilizing the improved aluminum chlorhydrate as prepared by the process of this invention.

### BACKGROUND OF THE INVENTION

Basic aluminum halides (also referred to as aluminum halohydrates) have long been known to possess antiperspirant activity. These antiperspirant compositions are available in the form of polymeric compositions having the empirical formula:

$$Al_2(OH)_{6-y}X_y$$

wherein X is chlorine, bromine or iodine and y has a numerical value from about 0.7 to about 3. However, it is only in recent studies, as described in U.S. Patent No. 4,359,456 (the '456 patent), that it has been shown by size exclusion chromatography that basic aluminum halides are composed of individual polymer bands which pertain to different molecular weight groups of the compound. In these studies of basic aluminum halides obtained by conventional methods of preparation it was shown that it can further be broken down from high molecular weight polymers into larger amounts of lower molecular weight polymers by diluting concentrated aqueous solutions thereof to lower aqueous concentrations and treating with heat and or aging at room temperature to produce more effective antiperspirants as shown in sweat reduction panel studies.

The '456 patent describes processes for the preparation of improved antiperspirant compositions of aluminum halohydrates, which involve heating a 2.5 to 8.5% by weight, based on aluminum, of an aqueous solution of an aluminum halohydrate of the formula:

$$Al_2(OH)_{6-y}X_y$$

where X and y are as defined above, at a temperature of 50 to 140°C for a period of time to impart to the aluminum product certain desired properties in respect of size exclusion chromatogram test bands. The products thus obtained from these processes have good antiperspirant activity, but the processes do not provide compositions containing larger amounts of the lower molecular weight polymers with a narrow polydispersity which are believed to possess greater antiperspirant activity.

In addition to the '456 patent, processes for the preparation of antiperspirant basic aluminum halides are shown in U.S. patents nos. 3,507,896; 3,891,745; 3,904,741; 4,038,373 and 4,053,570. However, none of these patents disclose polymeric compositions possessing the desired amounts of the lower molecular weight polymers as measured by the size exclusion chromatogram test band.

Complexes of zirconyl hydroxyl chloride and aluminum chlorhydrate are known in the art as having antiperspirant activity as disclosed in Great Britain Patent No. 2,144,992, published March 20, 1985, entitled "ANTIPERSPIRANTS". The product is prepared by heating a 2-20% solution to at least 50°C until a ratio of the heights of peaks 4 to 3 as measured by gel permeation chromatography exceeds 2:1. Complexes of the Zr/Al compounds with amino acids are also known in the art. While these compounds contain lower molecular weight polymers to increase efficacy they also have a wide polydispersity, a high form of aluminum to zirconium glycinate complexes and a low cationic charge. This is evidenced from higher molecular weight polymers found in peaks (1 + 2) as shown in the U.K. Patent No. 2,144,992 referred to above.

### SUMMARY OF THE INVENTION

It has surprisingly been found that polymeric basic aluminum halides having the empirical formula:

$$Al_2(OH)_{6-y}X_y \cdot nH_2O$$

wherein y has a numerical value from about 0.7 to about 3, X is chlorine, bromine or iodine; n is a numeral from about 0.8 to about 4.0 and the polymer distribution as characterized by size exclusion chromatogram test is: (a) 100% of the aluminum containing polymers are found in bands II, III and IV, and (b) band III contains at least 25% of the polymers, can be prepared by reacting an aluminum metal with a halogen compound having the formula HX were X is as previously defined, while maintaining the temperature of the reaction mixture at about 50°C to about 100°C. The aluminum metal is preferably in the form of pellets or powder.

The amount of water used is such as to have the final concentration of the polymer solution, in percent by weight, in the range of about 8 to about 35%, preferably about 8 to about 25%, more preferably about 15 to about 25%, and most preferably from about 17 to about 22% by weight. The reaction temperatures are preferably in the range of about 95 to about 100°C.

It has been found that an improved aluminum chlorhydrate/zirconyl hydroxychloride complex can be prepared by reacting the foregoing aluminum chlorhydrate of this invention with the zirconyl hydroxychloride and a neutral amino acid such as glycine. The aluminum chlorhydrate/zirconyl hydroxyhalide complex of this invention is characterized in that the peak height ratio of peak 4 to peak 3 is about 0.5 to about 1.8:1 and the peaks (1+2) content of the complex is less than 4% of the aluminum containing polymer distribution by weight.

## BRIEF DESCRIPTION OF THE DRAWINGS

Figure 1 is a chromatogram of a product made by a known conventional method as described in Example 1.

Figures 2 to 4 are chromatograms of products as they form during various states of the process of this invention.

Figures 5A-5C are chromatograms for aluminum chlorhydrates prepared using the method of U.S. Patent No. 3,891,745.

Figures 6A-6C are chromatograms for conventional Aluminum Chlorhydrate and Activated Aluminum Chlorhydrate prepared by methods and the process of this invention.

Figures 7 and 8 are chromatograms of aluminum-zirconium glycinates prepared by a prior art process.

Figures 9 and 10 are chromatograms of the aluminum-zirconium glycinates of this invention.

Figures 11 and 12 are chromatograms of aluminum-zirconium glycinates prepared by the method of U. K. Patent No. 2,144,992.

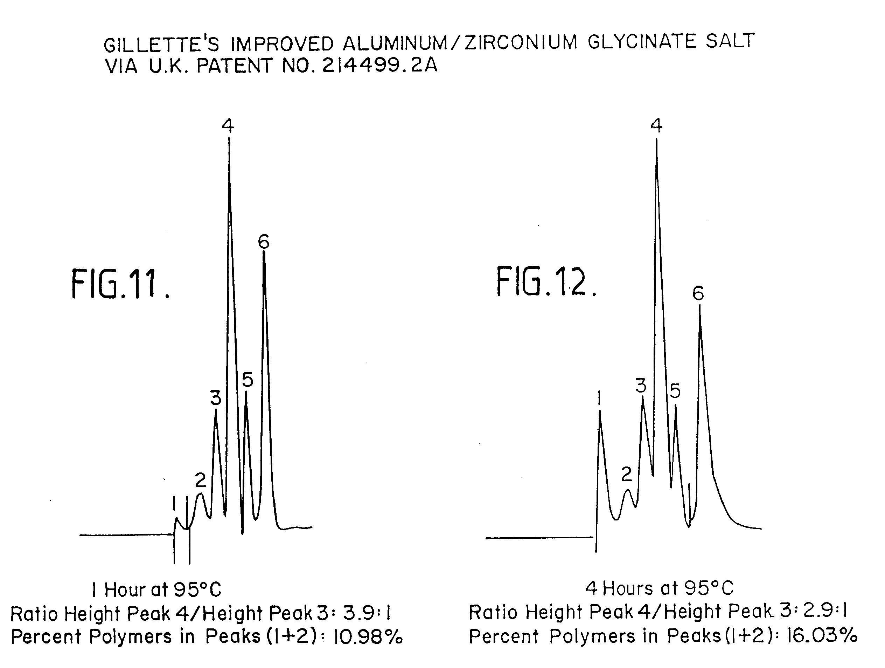

Figures 11 and 12 are chromatograms of the aluminum-zirconium glycinates of this invention.

Figures 13 and 14 are chromatograms of aluminum-zirconium glycinates prepared by the method of U. K. Patent No. 2,144,992.

## DETAILED DESCRIPTION OF THE INVENTION

This invention relates to an improved method for preparing an aluminum chlorhydrate having improved antiperspirant activity as determined by the existence of a band III component of polymer of at least 25 wt. %. The product of this invention is substantially free of any Band I component. Additionally, this invention relates to a method of making a complex of the aforementioned improved aluminum chlorhydrate and a zirconyl hydroxyhalide.

### Preparation of Aluminum Chlorhydrate

While the aluminum chlorhydrate product of this invention can be defined as having the empirical formula $Al_2(OH)_{6-y}X_y$, where y is 0.7 to 3 and X is Cl, Br or I, it will be understood that the aluminum halohydrate of this invention has associated with it both free water and coordinated water. The empirical formula showing this water is $Al_2(OH)_{6-y}X_y \rho nH_2O$, where y and X are as previously defined and n has a numerical value of about 0.8 to about 4; preferably about 1 to about 3.5; more preferably about 2 to about 3. Approximately 85 wt. % of the water is coordinated water as contrasted with conventional Aluminum Chlorhydrates which contain about 60% coordinated water.

The process comprises reacting metallic aluminum in the form of pellets, powder, chips or bars with a hydrohalogen acid of the formula HX, where X is chlorine, bromine or iodine. Preferably the acid is HCl.

While the reaction can be carried out at a temperature of about 50°C to about 100°C, it is preferred that the reaction is carried out at about 80°C to about 100°C; more preferably at about 90°C to about 100°C; most preferably at about 95°C to 100°C, e.g., about 96°C to about 98°C. The reaction is carried out in the absence of reflux conditions. Refluxing can result in reduced formation of the Band III component, and will result in the formation of pre-Band I high molecular weight polymers. However, it is within the

3

scope of this invention to utilize a condenser to condense and return water evaporated during the process to the reaction vessel in order to maintain the proper concentration of reactants and product in the reaction mixture.

Successful practice of the invention is best achieved when the quantities of aluminum, water and acid are selected so as to result in an exotherm of at least 5°C. Preferably, about 10°C to about 20°C. The desired exotherm can be achieved by using a concentration of HCl such that the water/HCl solution formed is at least a 3 wt. % concentration of HCl in the water; preferably about 5 wt. % to about 8 wt %. HCl. It is not necessary to premix the water and HCl in order to commence the reaction. It is preferred that they be added separately. As used in the specification and claims with reference to HCl, the concentration indicated means that concentration which a water/HCl solution would have if the quantity of water and acid utilized in the process were pre-mixed, notwithstanding the fact that pre-mixing is neither required nor preferred.

Generally, an excess of aluminum is used in carrying out the reaction process of this invention. This is so since aluminum must always be present throughout the reaction in order for the final product to be formed. However, where the aluminum is in a powdered form the reaction will go to completion using stoichiometric amounts of aluminum and HCl based on the anticipated formula of the product. For example, where the desired product is $Al_2(OH)_5Cl$ the HCl/Al ratio is determined based on that formula for aluminum chlorhydrate, and not on the stoichiometric amounts required to form aluminum chloride.

In carrying out the process of this invention the aluminum is preferably in pellet or powder form. While chemically pure aluminum can be utilized in the practice of this invention it is not preferred. The aluminum of choice contains trace amounts of iron or copper. The iron and copper catalyze the HX-Aluminum reaction, which results in substantial heat generation, thereby minimizing the amount of heating required to maintain the reaction mixture at the proper temperature. The preferred aluminum is a copper containing aluminum.

Although the concentration of iron in the aluminum can range from about 0.02 to about 0.25 wt. % in the preparation of concentrated solutions of aluminum chlorhydrate of the prior art, in the practice of this invention the iron concentration in the aluminum must be limited to about 0.02 to about 0.1 wt. %. Reactions which use aluminum having iron impurities of greater than 0.1 % result in aluminum salts having iron contents greater than the acceptable limits of the cosmetics trade. The concentration of copper in the aluminum can be about 0.005 to about 0.2 wt. %. Preferably, however, the copper content of the aluminum is about 0.005 to about 0.03 wt. %. It is of course within the scope of this invention to utilize aluminum metal containing both iron and copper.

A critical aspect of the process of this invention is the final concentration of aluminum halohydrate in the reaction mixture which must be maintained at a concentration in percent by weight in the range of about 8 to about 35%, preferably about 8 to about 25%, more preferably about 15 to about 25%, and most preferably from about 17 to about 22% by weight. Above 25 Wt.% the amount of Band III in the product diminishes where the halogen is chlorine. For example at a 35 % concentration the Band III component is reduced to about 20 % for an aluminum chlorhydrate. While the Band III levels will be higher where the halogen is bromine, though a desirable product, Aluminum Bromhydrate is not the most preferred product.

The process can be most advantageously practiced over the entire 8 to 25 wt. % range. It is preferred, however, that the minimum concentration be at least 15 wt. %. Below 15 % the solutions of product are cloudy. There appears to be a relationship between the cloudiness of the reaction solution and the development of higher molecular weight species found prior to Band II in the chromatographic distribution. When reactions are carried out in solutions having a concentration of less than 15 %, the development of cloudiness can be avoided by reducing the reaction temperature and shortening the reaction time. Where the solution concentration is below 15% it is preferred that the reaction temperature is below 90°C and that the reaction time is less than 24 hours; more preferably the reaction temperature is about 70 to about 85°C, e.g., 80°C.

The polymer distribution achieved by the process of this invention is one of extremely narrow polydispersity, particularly when the final batch concentration of aluminum halohydrate falls within the range of 17-22% and the metal to halogen atomic ratios are about 1.00:1 to about 2.10:1. Preferably these ratios are about 1.50:1 to about 2.00:1; more preferably about 1.90:1 to about 2.00:1. Such products derived from the process of this invention can be converted to more stable polymer forms by the hot spray drying of the solution of aluminum Chlorhydrate to a dry powder utilizing conventional spray drying methods.

Spray drying is the preferred method of converting the hot aqueous basic aluminum salts of this invention to a stable powdered form. Other methods of drying such as tray drying or vacuum drying require longer periods of time for water evaporation. These drying techniques go through concentrated liquid phases, or reduced temperatures, both of which can result in a substantial loss in the Band III component and the widing of the polydispersity value of Band II, of the product of this invention. In carrying out the spray drying process the outlet drying temperature can be about 150°F (71°C) to about 275°F (135°C); preferably about 200°F (94°C) to about 240°F (116°C); more preferably about 210°F (101°C) to about 230°F (110°C). If the aluminum halohydrate solution is allowed to cool after completion of the reaction, and before spray drying a loss in the Band III component to a level of less than 20% results. It is preferred that the solution be filtered before spray drying.

The size exclusion chromatogram test was used to determine polymer distributions, contents and relative retention times of Bands I, II, III and IV on the samples of the compositions of this invention and samples of known compositions. This test is an analytic technique related to high performance liquid chromatogram (HPLC). In carrying out the tests a Waters Associates Model 510 pump, a U6K injector, a 401 refractive index detector, and a 730 data module were used for the HPLC instrumentation. Two micro Porasil 60 Å GPC columns 3.8 x 30 cm (Waters Cat. No. 84190) were used in the adsorption.

The directions for carrying out the test are as follows:

In preparing the mobile phase, pipette 2 ml. conc. nitric acid in a 1 l. volumetric flask containing distilled water, dilute to mark and mix. New columns should be conditioned with this mobile phase at least three hours prior to sample testing. Turn pump on to 1.0 ml/min., flush the reference side of the refractive index cell several minutes and switch to sample side. Referring to the operator manual, zero in the R.I. detector and set the attenuation to 16X. Also set the 730 data module to the following parameter values:

| Parameter No. | Description | Value |
|---|---|---|
| 2 | Chart Speed | 0.6 (cm./min.) |
| 3 | Plot Mode | 0 (Off) |
| 4 | Pen 2 | 0 (Off) |
| 5 | Pen 1 | 10 |
| 7 | Auto Zero | 0 (Off) |
| 8 | L.C. Mode | 1 (Yes) |
| 9 | Calibration | 0 (Analysis) |
| 20 | Auto Parameters | 0 (Off) |
| 21 | Peak Width | 7 |
| 22 | Noise Rejection | 2,000 |
| 23 | Area Rejection | 1,000 |
| 24 | Run/Stop | 6.5 (Min.) |
| 33 | Report % results | 1 (Yes) |
| 46 | Flow Rate | 1.0 (ml./min.) |
| 47 | Pressure | Column Pressure |
| 48 | Detector/Attenuation | 401/016 |
| 63 | Report Percent Only | 1001 |

The analytical procedure is as follows:

Pipette 0.2 ml. 12 M hydrochloric acid into a 25 ml volumetric flask containing distilled water, dilute to mark and mix.

After the detector and columns have reached equilibrium as seen by the stability of the response on parameter 51, set parameter 51 to read 5,000 - 10,000 by turning the optical zero knob on the detector, being certain that operating temperatures within the room remain constant since the slightest change in the temperatures will be sensed by the R.I. detector which will create a base line drift.

Inject a 15 ml. sample of 0.1N hydrochloric acid standard and observe its retention time (the retention time in this analytical test was found to be 5.70 minutes). Set parameters 81 and 82 to retention time values off 5.40 and 6.00 minutes which will inhibit and resume integration without integrating the hydrochloric acid band itself which contains no aluminum polymers.

Dilute all basic aluminum halides to approximately a 10% active level with distilled water, filter the sample through a 0.45 m filter and inject a 3.0 ml sample for the test. The chromatogram will show which aluminum containing polymer bands are present, the retention times of each band and their calculated percentages.

Calculation:

$$\%\text{Band to be determined} = \frac{(\text{Area Percent of band to be determined})}{\text{Total Area Percent of Al containing bands}}$$

It is known that during stages of basic aluminum halides synthesis via conventional methods of preparation, higher molecular weight polymers, pertaining to aluminum polymers of the Band I range, are developed and their percent composition against the total polymer content increases with increasing metal to chloride atomic ratios. Table I shows the percent of Band I polymers found at various reaction states of aluminum chlorhydrate preparation when prepared by the conventional method.

TABLE I

| Aluminum/Chloride Atomic Ratio | % Band I Aluminum Polymers |
|---|---|
| 1.32:1 | 15.9 |
| 1.82:1 | 17.0 |
| 1.93:1 | 37.2 |

In addition to the formation of Band I, it is also known that basic aluminum halides produced via conventional methods of synthesis contain lower amounts of Band III than those amounts found using the process of this invention.

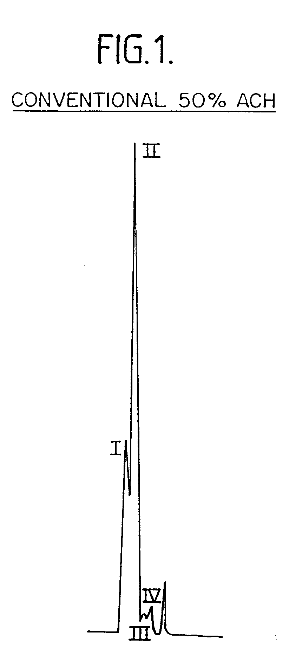

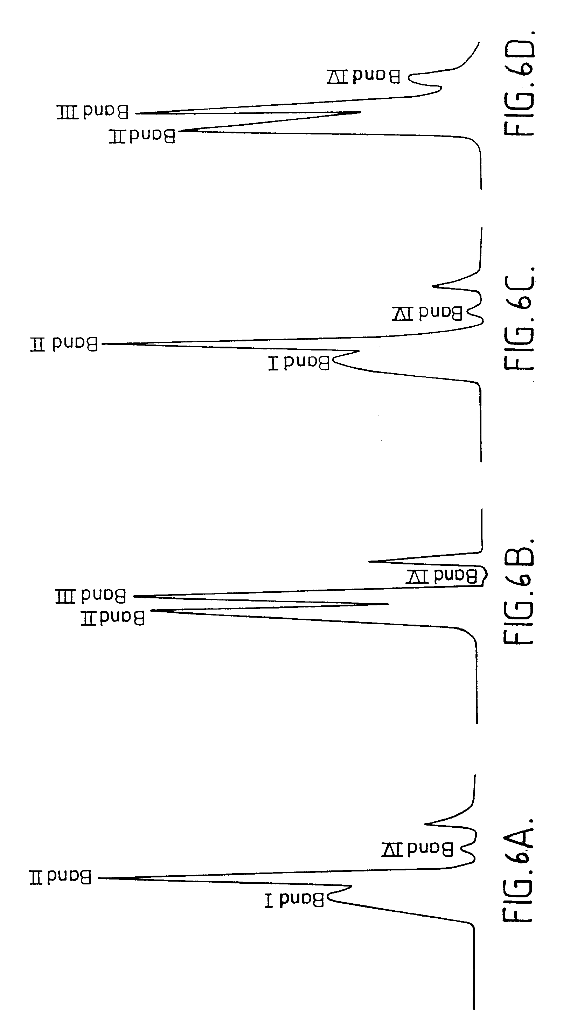

The advantages of the instant invention will be more readily appreciated by reference to the following examples and the drawings. The examples are intended to be illustrative of the invention and in no way limit the scope of this invention. In the drawings Figures 1 to 6 illustrate chromatograms of the product showing the four (4) typical aluminum polymer bands.

## EXAMPLE 1

Aluminum metal was reacted with hydrochloric acid and water, where the amount of water was stoichiometrically controlled so that the final reaction product contained a (water + hydroxyl)/aluminum molar ratio of about 9.7:1 and the active concentration was about 50%, as described below.

In a 250 ml glass reaction flask, equipped with a reflux condenser and thermometer, 30 g. of granulated aluminum was reacted with 125.8 grams of deionized water and 52 grams of 20 Baume hydrochloric acid. The batch was heated to 98°C until nearly all the aluminum was in solution and the aluminum to chloride atomic ratio was determined by analysis to be 2.00:1. The resulting 50% solution was filtered and its polymer composition was determined by the size exclusion chromatogram test previously described. The chromatogram in Figure 1 shows four typical aluminum containing polymer bands with relative retention times calculated with respect to the retention time of hydrochloric acid. Table II shows the retention times, relative retention times and the percent of the total aluminum polymers found in each band.

TABLE II

| Band | RI (Min.) | RRT | AL Polymer |
|---|---|---|---|
| I | 3.72 | 0.65 | 39.23 |
| II | 4.08 | 0.72 | 54.98 |
| III | 4.38 | 0.77 | 2.95 |
| IV | 4.89 | 0.86 | 2.85 |

The last unintegrated band in Figure 1 is that of hydrochloric acid which exists as free acid to some degree in all basic aluminum chlorides. In accordance with the test procedure described in the invention this peak was eluted at 5.7 minutes and it is this retention time that is used as the basis in calculating relative retention times of all other bands. The range of relative retention times for purposes of the invention has been defined as shown in Table III.

TABLE III

| Band No. | Relative Retention Time Range |
|----------|-------------------------------|
| Band I | 0.62 - 0.70 |
| Band II | 0.71 - 0.75 |
| Band III | 0.76 - 0.82 |
| Band IV | 0.83 - 0.97 |

The 50 % solution obtained in this example is a standard product in the industry and can be marketed as such or further processed to a basic aluminum chloride powder through common techniques such as spray drying, vacuum drying, etc.

Examples 2 to 5 illustrate the process of this invention.

## EXAMPLE 2

(A) A 20% solution of aluminum chlorhydrate was prepared by reacting 2.4 kg. of granulated aluminum, 12.5 kg. of distilled $H_2O$ and 4.25 kg. 20 Baume hydrochloric acid in a 50 liter reaction flask. During the exotherm of the reaction an additional 21.47 kg. of distilled $H_2O$ was charged. The temperature of the batch was maintained at 98°C through the oxidation reduction reaction for 72 hours. A sample was taken, filtered and tested for polymer composition using the size exclusion chromatography method previously described. The sample was also analyzed for percent aluminum, percent chloride and aluminum/chloride atomic ratio.

(B) The batch was filtered hot and immediately spray dried to a stable powder form using a No. 2 fluid nozzle on a conical bottom concurrent flow lab dryer at 320°F inlet, 160 °F, outlet and 95 PSI nozzle pressure. 7.15 kg. of an off white fine crystalline powder was obtained at the cyclone outlet which was also analyzed for percent Band III, percent polymer composition, aluminum, chloride and aluminum/chloride atomic ratio. The results for the 20% aluminum chlorhydrate and the spray dried powder are shown below.

TABLE IV

|  | 20% Solution | Spray Dried Powder |
|--------------|:------------:|:------------------:|
| % Aluminum | 5.00 | 24.75 |
| % Chloride | 3.38 | 16.78 |
| Al/Cl ratio 1.94:1 | | |
| % Band I | 0.0 | 0.0 |
| % Band II | 59.1 | 60.6 |
| % Band III | 39.6 | 38.1 |
| % Band IV | 1.3 | 1.3 |

## EXAMPLE 3

An 8% solution of aluminum sesquichlorhydrate was prepared by reacting aluminum powder with hydrochloric acid and water as follows: 50 grams of aluminum powder and 200 grams deionized were charged to a 500 ml glass reaction flask, equipped with a thermometer and condenser. 21 grams of 31.45% hydrochloric acid was added and an immediate reaction was observed. During the exotherm of the reaction an additional 172 grams of deionized water was added and the reaction mixture was maintained at about 80°C for 1 hour. A sample was taken, filtered and immediately tested for Band III, and polymer distribution, according to the chromatographic procedure previously described, as well as % $Al_2O_3$ and Al/Cl atomic ratio. The resulting 8% solution was shown to contain:

70.14% Band III

100.0% Polymer distribution in Bands II, III & IV

3.70% $Al_2O_3$

1.16:1 Al/Cl atomic ratio

## EXAMPLE 4

39.0 grams of granulated aluminum, 238 grams of deionized water and 61.0 grams of 20 Baume hydrochloric acid were charged to a 500 ml glass reaction flask equipped with a reflux condenser and thermometer. During the exotherm of the reaction additional heat was applied and the reaction was maintained at 98 C for 48 hours, filtered hot and analyzed. The resulting 25% aluminum sesquichlorhydrate solution contained:
25.5% Band III
100.0% Polymers in Band II, III & IV
11.40% $Al_2O_3$
1.86:1 Al/Cl atomic ratio

## EXAMPLE 5

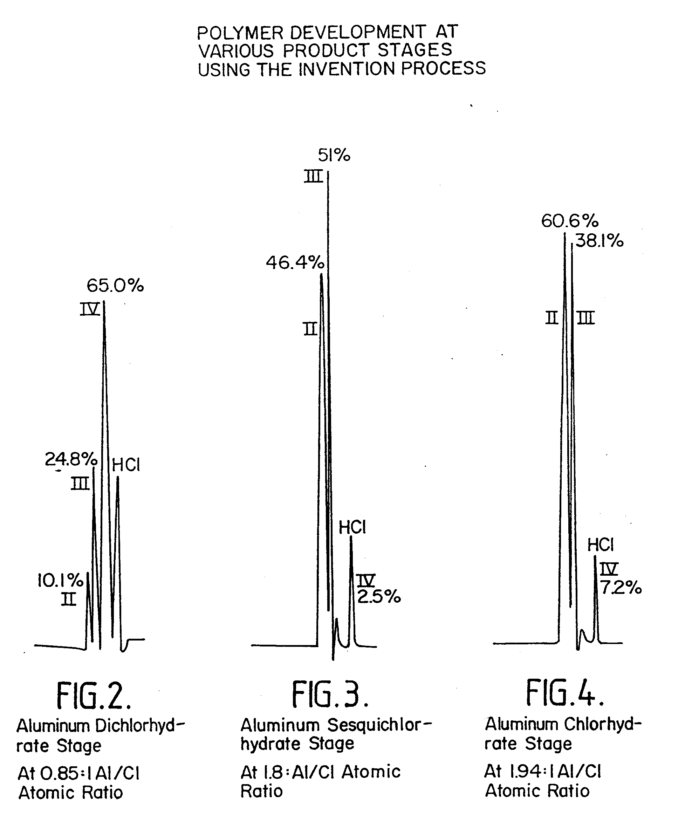

40 grams of granulated aluminum, 61.8 grams of 50% hydrobromic acid and 318 grams of deionized water were charged to a 500 ml reaction flask, equipped with a reflux condenser and thermometer. The contents were reacted at 90 C for 50 hours, filtered and tested for percent Band III, polymer distribution, % $Al_2O_3$ and aluminum to bromide atomic ratio. The resulting 25% aluminum bromhydrate solution contained:
65.5% Band III
100.0% Polymer distribution in Bands II, III & IV
9.7% $Al_2O_3$
1.90:1 Al/Br atomic ratio
In the products made by the process of this invention, Band I is not formed at any time during the reaction and Band III is formed in high percentages which results in the formation of lower polymeric forms of basic aluminum halides of narrow polydispersity. This is clearly shown in an examination of Figures 2 to 4.
In Figure 2, 65.0% of the aluminum polymers in Band IV exist in a very low molecular weight low basic form which together with the free hydrochloric acid (unintegrated band) are converted into Bands II and III as the reaction proceeds to the right.
Figure 3 shows that nearly all of Band IV and half of the free acid from Figure 2 have further reacted and converted into Bands II and III.
Figure 4 shows the completion of the invention process where half of Band IV, 25% of free acid and 20% of Band III which existed in the sesquichlorhydrate stage in Figure 3 have now been converted to Band II.
Table V shows the polymer characteristics of two aluminum basic halides prepared at 50% concentration (conventional reaction method) and two aluminum basic halides prepared at 20% concentrations using the process of this invention.

TABLE V

| Basic Aluminum Halide | Metal/Chloride Atomic Ratio | Band No. | | | |
|---|---|---|---|---|---|
| | | I | II | III | IV |
| 50% Alum. Chlorhydrate Solution | 1.99:1 | 41.8 | 51.7 | 1.0 | 3.5 |
| 50% Alum. Bromhydrate Solution | 1.90:1 | 7.3 | 74.8 | 15.4 | 2.5 |
| 20% Alum. Chlorhydrate Solution | 1.94:1 | 0 | 56.7 | 40.2 | 3.1 |
| 20% Alum. Bromhydrate Solution | 1.92:1 | 0 | 26.7 | 69.6 | 3.7 |

Examples 6 & 7 are outside the scope of the invention.

## EXAMPLE 6

U.S. Patent No. 3,891,745 assigned to Dynamit Nobel discloses a process for preparing aluminum chlorhydrate from HCl and Aluminum. From the disclosure and claims, and in particular the reference to pH, it is apparent that the reaction is carried out at the typical concentrated solution of the prior art.

Furthermore, the example states that the end product was collected in the form of a 47% by weight solution. A scaled down *in vitro* experiment was conducted to reproduce the Dynamit Nobel process.

120 grams of Aluminum granules were placed in a 300 mm chromatography column fitted with a stop cock and wrapped in heating tape in the lower 2/3 section of the column. The top of the column was fitted with a water cooled condenser.

100 ml of 11% hydrochloric acid was added to the column and reaction was immediately observed. Column temperature at the lower section was maintained at about 80°C. The reaction was allowed to proceed for three hours. Before taking the first sample from the bottom valve. The contents were allowed to elute at one drop per minute from the bottom while adding another 100 ml. of 11% HCl into the top at the same rate. Two more samples were taken at 6 and 8 hours after the reaction commenced. The samples were analyzed for % Al, % Cl and Al/Cl ratio using the above described methods. The results are as follows:

TABLE VI

| Component (Chromatogram) | 3 Hr. Sample (Figure 7A) | 6 Hr. Sample (Figure 7B) | 8 Hr. Sample (Figure 7C) |
|---|---|---|---|
| % Aluminum | 2.82 | 6.68 | 12.37 |
| % Chloride | 10.26 | 10.20 | 9.71 |
| Al/Cl Ratio | 0.36:1 | 0.86:1 | 1.67:1 |
| % Active Sol. | 25 | 35 | 55 |

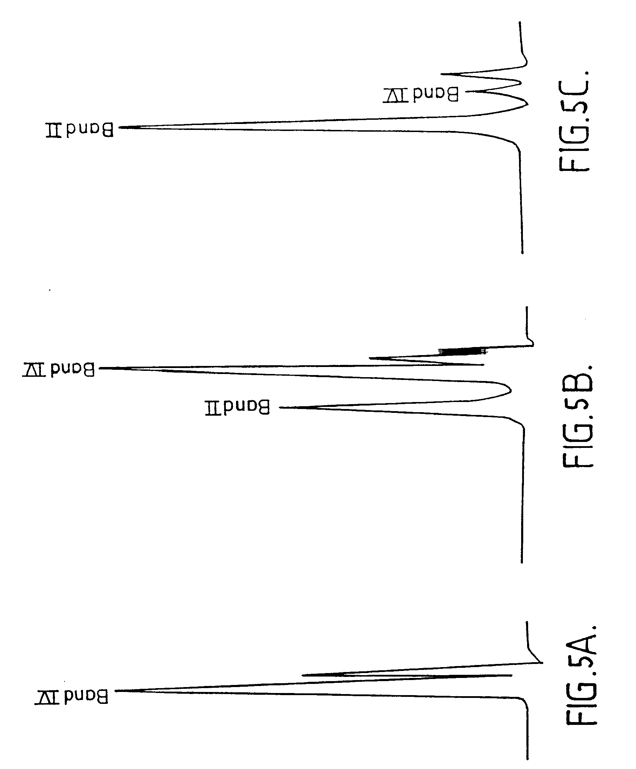

Figure 5 shows the size exclusion chromatography chromatograms for each sample. It is noted that the 3 hour sample (Figure 5A) exhibited only a Band IV while the 6 and 8 hour samples, Figure 5B & 5C respectively, exhibited Bands II & IV. None of the samples exhibited a Band III. Hence the product of the U.S. 3,891,745 patent is not the product of this invention. Therefore the process of this invention is a different process than that of the Dynamit Nobel '745 patent.

## EXAMPLE 7

39.0 grams of granulated aluminum, 294 grams of deionized water and 76.0 grams of 20 Baume hydrochloric acid were charged to a 500 ml glass reaction flask equipped with a reflux condenser and thermometer. During the exotherm of the reaction additional heat was applied and the batch was mildly refluxed for 48 hours, filtered hot and analyzed as described in Example 4. The resulting 35% aluminum sesquichlorhydrate solution contained:

20.01% Band III
100.0% Polymers in Band II, III & IV
15.80% $Al_2O_3$
1.86:1 Al/Cl atomic ratio

Example 8 demonstrates that the product of this invention is different from both conventional aluminum chlorhydrate and the activated chlorhydrate of the prior art.

## EXAMPLE 8

In comparison studies of the aluminum chlorhydrate of this invention and prior art compounds significant differences in viscosity were found. Blends of aluminum chlorhydrate were prepared with bentonite clay in formulations of the type used in cosmetic antiperspirants presently being marketed. Two blending techniques were used. Though each technique resulted in different numerical values, the trend toward higher viscosities for the blends utilizing the aluminum chlorhydrate of this invention was consistent.

DISPERSION METHOD I

Formulation:

30% Aluminum Chlorhydrate (ACH)
30% Bentone/Silicone Gel
40% Volatile Silicone

9

The Gel and volatile silicone were homogenized together and the ACH subsequently dispersed into the mixture. The Brookfield Viscosity of the dispersion was measured after 24 hours using a No. TB spindle for the lower viscosity (below 900,000 cp) solutions and a TF spindle for the higher viscosity solutions. The viscosity comparisons are shown in Table VII. A homogenized Gel and volatile silicone mixture without ACH was used as a control.

TABLE VII

| BLEND ACH COMPONENT | | Brookfield Viscosity (cp) | CHROMATOGRAM FIGURE |
|---|---|---|---|
| A. | Westwood Chemical Company conventional ACH made by HCl process. | 49,000 | Figure 8A |
| B. | Activated ACH of this invention | 66,000 | Figure 8B |
| C. | Reheis Conventional ACH via AlCl$_3$ Reaction Process | 25,000 | Figure 8C |
| | Control | 69,760 | |

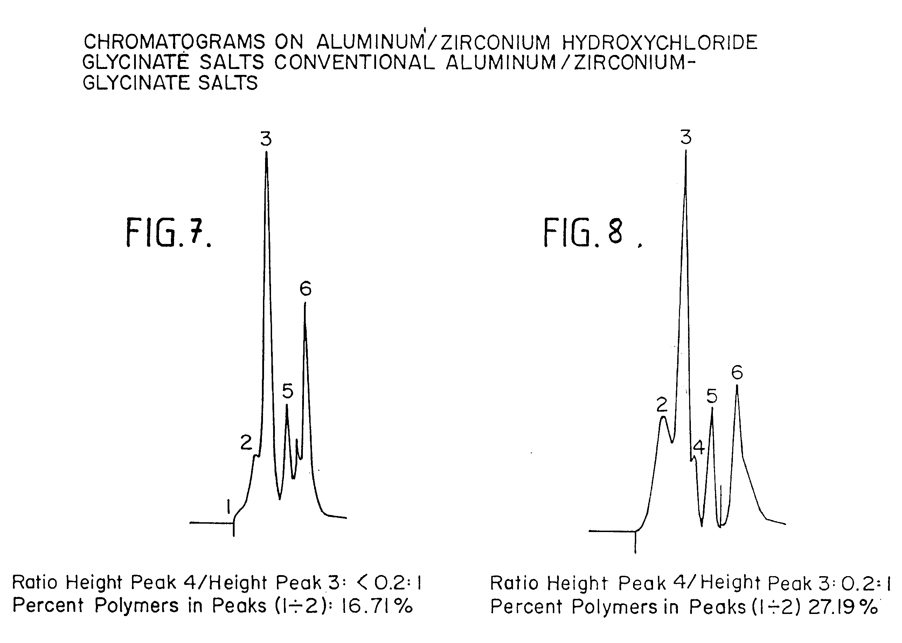

It will be noted that all samples containing ACH have viscosities below that of the ACH free control. Blend B which contains the ACH of this invention has a substantially higher viscosity than any of the prior art compositions. Chromatographies of the compositions are shown in Figure 8. Blends A and C which are conventional ACH compositions have substantially identical chromatographs showing Band I, II and IV peaks but no Band III peak. While the differences in the B and C chromatographs appear to be minor, they are significant, and there significance is evidenced by the differences in the behavior of the products in formulations as indicated by viscosity differences.

DISPERSION METHOD II

The experiment of Dispersion Method I was repeated using the same formulation except that all three component of the blend were homogenized in one step. The viscosity results are shown in TABLE VIII.

TABLE VIII

| BLEND ACH COMPONENT | | Brookfield Viscosity (cp) | CHROMATOGRAM FIGURE |
|---|---|---|---|
| A. | Westwood Chemical Company conventional ACH made by HCl process. | 2,000,000 | Figure 8A |
| B. | Activated ACH of this invention | 900,000 | Figure 8A |
| C. | Reheis Conventional ACH via AlCl$_3$ Reaction Process | 1,750,000 | Figure 8C |
| | Control | 200,000 | |

The invention provides a reaction process for the preparation of polymeric basic aluminum halides where Band I is not formed, and at least 25% of the composition is found in Band III. A narrow distribution of polymers of low molecular weight is obtained where 100% of the aluminum containing polymers fall within chromatographic Bands II, III & IV as defined by the relative retention time ranges using the test method described within the invention. In the process of this invention no Band I component ever exists, unlike prior art processes which begin with a product containing Band I and convert the starting material to a high band III component product.

**Preparation of Aluminum Halohydrate/Zirconyl Hydroxychloride Complex**

The aluminum halohydrate/zirconyl hydroxy chloride complex of this invention is prepared by mixing solutions of the foregoing novel aluminum halohydrate with a solution of irconyl hydroxychloride. For the purpose of clarity the aluminum halohydrate solution will be referred to as Component A and the zirconyl hydroxychloride is referred to as component B. Either or both of these solutions may contain a neutral amino acid to aid in buffering and to prevent gelation in the final composition. The two components are contacted for less than two hours at 50°C to about 100°C and immediately dried to a powder. THe product so formed when subjected to gel permeation chromatography is found to contain a peak height ration of peak 4 to peak 3 of about 0.5 to 1.8:1, and a peaks (1 + 2) totaling less than 4% of the aluminum containing polymer composition. Preferably the peak 4 to peak 3 height ratio is about 0.5 to about 1.5; more preferably about 0.8 to about 0.8 to about 1.45. The Al/ZR complex of this invention is prepared in the following manner:

## COMPONENT B

In accordance with the invention there is provided a process for preparing a Al/Zr complex from a zirconyl hydroxychloride having the empirical formula:

$$ZrO(OH)_xCl_{2-x}$$

wherein x has a numerical value of 0-1.5. The metal to chloride ratio of the hydroxychloride is preferably about 0.6 to 1.2:1. In the practice of this invention a solution of the hydroxychloride is prepared and reacted with a solution of Component A.

The amount of water used in preparing the zirconyl hydroxychloride solution (Component B) is such that the percentage by weight of hydroxychloride in solution is about 20 to about 55%. In a preferred embodiment the concentration of hydroxychloride is about 50-55% by weight. By using the higher concentration range, the rate of complexing between the amino acid and the basic zirconium chloride is greatly retarded, thus allowing for a lower form of complex in the final product. The solution is heated to a temperature of 50°C to about 100°C and a neutral amino acid, such as glycine can be added to the solution to form a zirconyl chloride-amino acid complex. The atomic ratio of amino acid to zirconium is in the range of 0 - 3.0:1. Component A can then be added to the solution. Component A comprises a solution containing about 8 to about 35 wt% aluminum halohydrate prepared by the aforedescribed HCl/Al reaction process.

Upon mixing of the two solutions there is formed an aluminum-zirconium hydroxy halide-amino acid complex of the empirical formula:

$$(Al_2(OH)_{6-y}X_y)_a(ZrO(OH)_xCl_{2-x})_b\text{neutral amino acid}$$

wherein x has a numerical value about of 0 - 1.5, X is Cl, Br I, and y has a numerical value of about 0.7 to about 3.0. The values of a and b are selected so that the aluminum/zirconium atomic ratio is preferably about 2.0 to about 8.0. The metal to amino acid ratio is preferably about 1.0 to about 10:1..

In preparing the Al/Zr complex preferably an 8 - 25 wt. % solution of aluminum halohydrate of this invention (Component A) at a temperature of about 50°C to about 100°C is blended with a 20 - 55 wt. % solution of zirconium hydroxychloride (component B) at a temperature of about 50-100°C. The preferred halohydrate is the chlorhydrate. In a preferred embodiment a solution containing about 17 to about 22 wt. % halohydrate at a temperature of about 95 - 97°C is blended with a 50 - 55 wt. % zirconyl hydroxy chloride solution at a temperature of about 50 - 55°C to form a solution at about 85°C. Either or both of the starting solutions may contain amino acid prior to the mixing of the two solutions.

Contact time of components A and B should not exceed two hours. Longer contact times can result in the formation of higher polymer forms found in peaks (1 + 2) by gel permeation chromatography. preferably the contact time is about 30 minutes to one hour; more preferably the contact time about 15 to about 30 minutes; most preferably the contact time is less than 30 minutes, e.g., 10 to 25 minutes. than 30 minutes. The combined solutions are then immediately dried to a powdered form. The preferred method of drying is spray drying using conventional spray drying techniques. Spray drying avoids prolonged heating of the solution as may occur during a tray drying operation.

The product formed by the process of this invention, when analyzed by gel permeation chromatography exhibits a peak 4 to peak 3 ratio of about 0.5 to 1.8:1. Peaks (1 + 2) contain less than 4% by weight of the aluminum containing polymers. The shorter the contact time of the two solutions, the less of the undesirable higher molecular weight polymers are formed. These higher molecular weight polymers appear in peaks (1 + 2). Similarly, the shorter contact time results in a lower amount of amino acid complex formed. As a result the products of this invention have a higher cationic charge than prior art composition, and consequently these salts are more efficacious as antiperspirants.

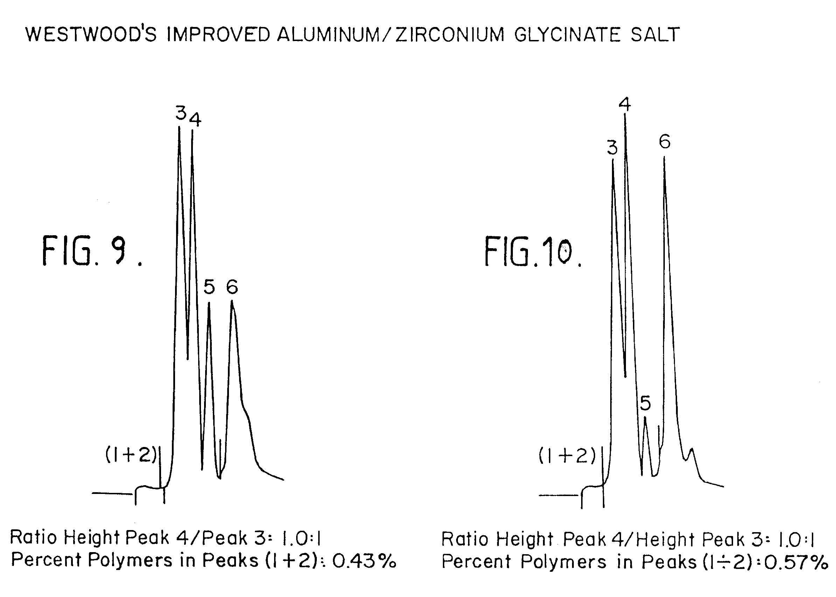

The following examples serve to illustrate the process of this invention and are not intended to be limiting. In the drawings Figures 9 to 14 illustrate the chromatograms of the Al/Zr complex showing the peak 4 to peak 3 ratio.

Figures 9 and 10 are chromatographs of aluminum-zirconium glycinates prepared by a prior art conventional process. Figures 11 and 12 are chromatograms of the Al/Zr glycinate complex of this invention and illustrate that the peak 4 to peak 3 ratio is in the range of 0.5 to 1.8:1. Figures 13 and 14 are chromatograms of an aluminum-zirconium glycinate salt prepared according to U. K. patent No. 2,144,992, by heating a10% solution of complex to 97°C for one and four hours respectively.

## EXAMPLE 9

### STEP A - Preparation of Component A

Three liters of deionized water and 0.576 Kg. of aluminum metal were charged to a 12 liter reaction flask. 1.02 Kg. of 20 degree Baume hydrochloric acid was charged to the flask, and an immediate exothermic reaction was observed. During the initial stage of the reaction an additional 5.15 liters of water was added, and the batch temperature was held at about 95-97°C for 72 hours. A solution containing about 20 wt. % of aluminum chlorhydrate was withdrawn from the flask and filtered hot while maintaining the temperature at 97°C. A sample of the solution was analyzed and found to contain 5.26% aluminum and 3.58% chlorine. The product was a 5/6 basic aluminum chlorhydrate to which was added 0.20 Kg. of glycine to form an aluminum chlorhydrate-glycinate complex. (Component A).

### STEP B; Preparation of Zirconium hydroxychloride

1.95 Kg. of a 50% zirconium hydroxychloride solution with a metal to chloride atomic ratio of 0.96:1 was charged to a 4 liter flask and heated to 50°C. 0.20 Kg. of glycine was added to form a zirconyl hydroxychloride gly complex (component B).

### Preparation of Al/Zr Glycinate Complex

2.65 Kg. of Component A at 97 C was charged to a flask containing 0.65 Kg. of component B at 50°C. The combined solution was transferred to a spray dryer feed pot where the temperature was maintained at 85°C. The solution was immediately spray dried in a conical bottom spray drier at 400°F inlet and 215°F outlet temperatures. The average contact time of the feed solutions was 15 minutes. Three consecutive batches of solution were prepared in order to minimize contact time of Components A and B.

2.57 Kg. of aluminum zirconium hydroxychloride glycinate complex was recovered, analyzed and found to contain 14.9% zirconium and 14.3% aluminum. Based on gel permeation chromatography of a sample the product had a peak 4 to peak 3 ratio of 0.95:1 with 0.7 wt % of the aluminum containing polymers in peaks (1+2) as shown in Figure 11. A comparison of the chromatograms of the product of this invention with chromatograms of product prepared by the method of U.K. '992 (Figures 13 and 14) readily shows the graphic difference between the peak 4 to peak 3 ratio as well as the peaks (1+2 content. Not wishing to be bound by theory the improvement in the product of this invention is believed to be attributable to the peak 4 to peak 3 ratio (0.5 to 1.8:1) and the and narrow polydispersity of the product as Illustrated by little polymer being found in peaks 1 and 2.

## EXAMPLE 10

1.44 Kg. of aluminum granular ingots were added to a 50 liter round bottom flask with 5.08 Kg. of deionized water. 2.61 Kg. of 20* baume HCl was added. An exothermic reaction ensued immediately. During the course of the reaction an additional 12.53 Kg. of deionized water was slowly added. The temperature was brought up to 98*C and held for 72 hours without refluxing. A condenser was used on the flask in order to maintain a constant volume of water in the reaction mixture. After 72 hours the aluminum ingots were removed. The resulting solution contained 20% aluminum chlorhydrate which upon analysis was found to contain 5.01% aluminum and 3.46% chloride with an aluminum/chloride ratio of 1.90:1. The solution temperature was maintained at 98*C. (Component A).

3.56 Kg. of zirconium basic carbonate was added to a reaction vessel containing 1.41 Kg. of 20 degree Baume hydrochloric acid and 10.66 Kg. of deionized water. An exothermic reaction was observed. When the reaction subsided the resulting solution was placed in a twelve liter round bottomed flask, and heated at 95*C for two hours without refluxing. A condenser was used to maintain water volume. 1.20 Kg. of glycine was added to form a zirconium hydroxychloride-glycine solution at 95*C without refluxing. An analysis of the solution showed 6,68% zirconium and 2.65% Chloride, a zirconium/chloride ratio of 0.98:1 and 7.06% glycine. The resulting 20% zirconyl hydroxychloride-gly was labeled Component B.

Component B at 95* was slowly added with stirring to Component A which was maintained at 98°C using an addition funnel on a 50 liter round bottom flask equipped with a condenser. The temperature was maintained at 98°C without refluxing. The HPLC Chromatogram taken at a contact time of 15 minutes showed a peak 4 to peak 3 band height ratio of 1.41:1 with less than 1% of the aluminum containing polymers in peaks (1 + 2). An analysis showed 2.93% aluminum, 2.71% zirconium, an aluminum/zirconium ratio of 3.65:1, 3.51% chloride and 2.87% glycine.

## EXAMPLE 11

1.44 Kg. aluminum granular ingots were added to a 50 liter round bottom flask with 5.08 Kg. deionized water and 2.61 Kg. 20 degree Baume hydrochloric acid. An exothermic reaction occurred. During the course of the reaction 12.53 Kg. deionized water was slowly added. The temperature was brought up to 98°C for 72 hours without refluxing. A condenser was used to maintain water volume. After 72 hours the aluminum ingots were removed and an analysis was performed which showed a 5.21% aluminum content, 3.57% chloride and an aluminum/chloride ratio of 1.92:1. 0.50 Kg. of glycine were added to the aluminum chlorhydrate with 0.24 kg. of 20 degree baume hydrochloric acid. The 20% basic aluminum sesquichlorhydrate-gly was maintained at 98°C and labeled Component A.

3.56 Kg. of zirconium basic carbonate was added to a reaction vessel containing 1.41 Kg. of 20 degree Baume hydrochloric acid and 10.66 Kg. of deionized water. An exothermic reaction was observed. When the reaction subsided the resulting solution was placed in a twelve liter round bottomed flask, and heated at 95°C for two hours without refluxing. A condenser was used to maintain water volume. 0.50 Kg. of glycine was added to form a zirconium hydroxychloride-glycine solution at 95°C without refluxing. The resulting 50% zirconium hydroxychloride-gly solution was allowed to cool to 50°C and was labeled Component B.

Component B at 50° was slowly added with stirring to Component A which was maintained at 98°C using an addition funnel on a 50 liter round bottom flask equipped with a condenser. The resulting temperature for the aluminum zirconium tetrachlorhydrex-gly solution was 84°C. The HPLC chromatogram taken after 15 minutes showed a peak 4 to peak 3 band height ratio of 1.00:1 with less than 1% of the aluminum containing polymers in peaks (1 + 2). An analysis showed 4.46% aluminum, 3.99% zirconium, an aluminum/zirconium ratio of 3.78:1, 4.64% chloride and 3.48% glycine.

## EXAMPLE 12

1.44 Kg. aluminum granular ingots were added to a 50 liter round bottom flask with 5.08 Kg. deionized water and 2.61 Kg. 20 degree Baume hydrochloric acid. An exothermic reaction occurred. During the course of the reaction 12.53 Kg. deionized water was slowly added. The temperature was brought up to 90°C for 72 hours without refluxing. A condenser was used to maintain water volume. After 72 hours the aluminum ingots were removed. A 20% aluminum chlorhydrate solution was recovered, and upon analysis was found to contain 5.18% aluminum, 3.53% chloride and an aluminum/chloride ratio of 1.93:1. 0.64 Kg. of glycine were added to the aluminum chlorhydrate solution. The basic aluminum chlorhydrate-gly was labeled Component A.

3.56 Kg. of zirconium basic carbonate was added to a reaction vessel containing 1.41 Kg. of 20 degree Baume hydrochloric acid. An exothermic reaction was observed. When the reaction subsided the resulting solution was placed in a twelve liter round bottomed flask, and heated at 95°C for two hours without refluxing. A condenser was used to maintain water volume. 0.64 Kg. of glycine was added to form a zirconium hydroxychloride-glycine solution which was allowed to cool to 30°C. An analysis showed 19.43% zirconium. 8.04% chloride, a zirconium/chloride ratio of 0.94:1 and 11.89% glycine. The resulting solution was labeled Component B.

Component B at 30° was slowly added with stirring to Component A which was maintained at 98°C using an addition funnel on a 50 liter round bottom flask equipped with a condenser. The resulting temperature for the aluminum zirconium trichlorhydrex-gly was 78°C. The HPLC chromatogram taken at a contact time of 15 minutes showed a peak 4 to peak 3 band height ratio of 0.90:1 with less than 1% of the aluminum containing polymers in peaks (1 + 2). An analysis showed 4.19% aluminum, 4.09% zirconium, an aluminum/zirconium ratio of 3.46:1, 4.87% chloride and 4.85% glycine.

## EXAMPLE 13

2.74 Kg. of aluminum chlorhydrate powder having 35.1% of its aluminum containing polymers in Band III was dissolved in 11.0 Kg. of deionized water at 95° to form a 20% solution of aluminum chlorhydrate. The temperature of the solution was maintained at 95°C without refluxing, and 0.43 Kg. of glycine was added with stirring. The basic aluminum chlorhydrate-gly was labeled Component A, and analyzed 4.96% aluminum, 3.26% chloride and 2.66% glycine. The aluminum/chloride ratio was found to be 1.98:1.

2.04 Kg. of zirconium basic carbonate was added to a reaction vessel containing 0.80 Kg. of 20 degree Baume hydrochloric acid. An exothermic reaction was observed. When the reaction subsided the resulting solution was placed in a twelve liter round bottomed flask, and heated at 95°C for two hours without refluxing. A condenser was used to maintain water volume. 0.43 Kg. of glycine was added while maintaining the temperature at 95° to form a zirconium hydroxychloride-glycine solution which was allowed to cool to 30°C. An analysis showed 17.78% zirconium. 7.40% chloride, a zirconium/chloride ratio of 0.93:1 and 11.11% glycine. The resulting solution was labeled Component B.

Component B at 30° was slowly added with stirring to Component A which was maintained at 95°C using an addition funnel on a 12 liter round bottom flask equipped with a condenser. The resulting temperature for the aluminum zirconium trichlorhydrex-gly was 77°C. The HPLC chromatogram taken at a contact time of 15 minutes showed a peak 4 to peak 3 band height ratio of 1.06:1 with 0.43% of the aluminum containing polymers in peaks (1 + 2). An analysis showed 4.62% aluminum, 4.25% zirconium, an aluminum/zirconium ratio of 3.68:1, 4.84% chloride and 5.67% glycine.

## EXAMPLE 14

10 grams of aluminum powder an 20 grams of aluminum granular ingots were reacted with 52 grams of HBr (47% active) and 333 grams of deionized water. The reaction temperature was maintained at 95°C for 24 hours. The solution was filtered and brought back to 95°C without refluxing. 3 grams of glycine were added to the aluminum bromhydrate solution and labeled Component A.

179 g. of zirconium basic carbonate was added to a one liter beaker with 68 g. of 20 degree Baume hydrochloric acid. An exothermic reaction was observed. When the reaction subsided the resulting solution was placed in a 500 ml. round bottomed flask, and heated at 95°C for two hours without refluxing. A condenser was used to maintain water volume. 15 g. of glycine was added while maintaining the temperature at 95°C to form a zirconium hydroxychloride-glycine solution which was allowed to cool to 30°C. The resulting solution was labeled Component B.

20 g. of aluminum chlorhydrate powder, which contained 36.4% of the aluminum containing polymer in Band III, was added to a 500 ml. round bottom flask with 85.7 grams of Component A and 14.3 grams of Component B. The contact temperature was 85°C which was maintained for two hours. The HPLC chromatogram take at initial contact of all of the components showed the peak 4 to peak 3 height ratio to be 0.91:1 with 0.65% of the aluminum containing polymers in peaks (1 + 2). After two hours at 85°C the HPLC chromatogram showed the peak 4 to peak 3 height ratio to be 0.50:1 with 0.50% of the product in peaks (1 + 2).

In addition to the features of the present invention described above, the following are also intended as preferred embodiments of the present invention:

In the process for preparing the aluminum halide having the empirical formula:

$$Al_2(OH)_{6-y}X_y \cdot nH_2O$$

a) the temperature at which the reaction of the aluminum with the hydrohalogen acid is carried out is preferably between about 70°C and about 100°C.

b) the hydrohalogen acid used is preferably HCl or HBr, most preferably HCl.

## Claims

1. A process for preparing a basic aluminum halide having the empirical formula:

$$Al_2(OH)_{6-y}X_y \cdot nH_2O$$

where n has a numerical value of about 0.8 to about 4, X is chlorine, bromine or iodine and y has a numerical value of about 0.7 to about 3, characterised in that it comprises:

(a) reacting aluminum metal with a hydrohalogen acid having the formula HX wherein X is chlorine, bromine or iodine at a temperature of about 50ºC to about 100ºC for about 1 to 100 hours, the concentration of the product in the solution being about 8 to about 35% by weight of the solution; and

(b) recovering the basic aluminum compound from the hot solution;

whereby the polymer distribution of the product formed as characterised by size exclusion chromatography is:

(c) 100% of the polymers are found in Bands II, III and IV, with substantially no part of the product found in Band I, and

(d) Band III contains at least 25% of the polymer.

2. A process according to Claim 1, characterised in that the aluminum is in the form of pellets or powder.

3. A process according to Claim 1 or Claim 2, characterised in that the aluminum contains copper in an amount of about 0.005 to about 0.03 wt. %.

4. A process according to Claim 1 or Claim 2, characterised in that the aluminum contains iron in an amount of about 0.02 to about 0.1 wt. %.

5. A process according to any of the preceding claims, characterised in that the aluminum is present in excess of the stoichiometric amount calculated on the acid utilized.

6. A process according to any of the preceding claims, characterised in that the aluminum halohydrate is recovered by spray drying the hot solution.

7. A process according to any of the preceding claims, characterised in that the concentration of product in the solution is about 8 to about 15 wt.%, the reaction temperature is maintained at about 75ºC to about 85ºC and the reaction time is less than 24 hrs.

8. A method for preparing an aluminum zirconium hydroxy halide glycinate complex having the empirical formula:

$$(Al_2(OH)_{6-y}X_y)_a(ZrO(OH)_xCl_{2-x})_b \text{Neutral Amino Acid}$$

wherein x has a numerical value from 0 to 1.5, y has a numerical value from 0.7 to 3.0, x is chlorine, bromine or iodine and a and b are selected so that the Al/Zr atomic ratio is about 2.0 to about 8.0, characterised in that it comprises:

(a) introducing an aqueous solution of Component A comprising 8 - 35% of a basic aluminum halide at a temperature of 45 to 100ºC and having the empirical formula:

$$Al_2(OH)_{6-y}X_y.nH_2O$$

wherein y has a numerical value from about 0.7 to about 3, X is chlorine, bromine or iodine, and n has a numerical value from about 0.8 to about 4, and whose polymer distribution as characterised by a size exclusion chromatography test has at least 25% of its polymers in Band III and 100% of the aluminum containing polymers in Bands II, III and IV;

(b) introducing an aqueous solution of Component B comprising a 20-55% solution of zirconyl hydroxychloride havingthe empirical formula:

$$ZrO(OH)_xCl_{2-x}$$

wherein x has a numerical value of from 0 to 1.5; a neutral amino acid being included in the solutions of either or both of Components A and B;

(c) mixing Component A and Component B at a temperature of from 50ºC to 100ºC for a time period of less than 2 hours to form a combined solution, thereby forming a complex of Component A and Component B and amino acid; and

(d) spray-drying the complex to a powder having a polymer distribution as characterised by the size exclusion chromatography test of:

   1) a peak height ratio of peak 4 to peak 3 of 0.5 to 1.8:1, and

   2) peaks (1 + 2) of less than 4% of the polymer distribution by weight.

**9.** A method according to Claim 8 characterised in that said mixing step (c) further comprises adding a neutral amino acid to Component A in an amount such that the atomic ratio of amino acid to aluminum is about 0 to about 3.0:1.

**10.** A method according to Claim 8 characterised in that said mixing step (c) further comprises adding a neutral amino acid to Component B in an amount such that the atomic ratio of amino acid to zirconium is about 0 to about 3.0:1.

**11.** A method according to any of Claims 8 to 10, characterised in that said temperature prior to mixing of Component A is about 85 to about 98ºC and the temperature of Component B is about 30 to about 50˚C.

**Patentansprüche**

**1.** Verfahren zur Herstellung eines basischen Aluminiumhalogenids der empirischen Formel

$$Al_2(OH)_{6-y}X_ynH_2O$$

in der n einen Zahlenwert von etwa 0,8 bis etwa 4, X Chlor, Brom oder Jod und y einen Zahlenwert von etwa 0,7 bis etwa 3 bedeutet, dadurch gekennzeichnet, daß es folgende Schritte umfaßt:

   (a) Umsetzung von Aluminiummetall mit einer Halogenwasserstoffsäure der Formel HX, in der X Chlor, Brom oder Jod bedeutet, bei einer Temperatur von etwa 50 bis etwa 100 °C für etwa 1 bis 100 Stunden, wobei die Konzentration des Produkts in der Lösung etwa 8 bis etwa 35 Gew.-%, bezogen auf die Lösung beträgt;

   (b) Gewinnung der basischen Aluminiumverbindung aus der heißen Lösung;

   wobei die Polymerverteilung des gebildeten Produkts, wie durch Größenexklusionschromatographie charakterisiert, gekennzeichnet ist durch:

   (c) 100 % des Polymers werden in den Banden II, III und IV gefunden, wobei praktisch kein Produkt in Bande I gefunden wird, und

   (d) Bande III enthält mindestens 25% des Polymers.

**2.** Verfahren nach Anspruch 1, dadurch gekennzeichnet, daß das Aluminium in Form von Pellets oder Pulver vorliegt.

**3.** Verfahren nach Anspruch 1 oder 2, dadurch gekennzeichnet, daß das Aluminium Kupfer in einer Menge von 0,005 bis 0,03 Gew.-% enthält.

**4.** Verfahren nach Anspruch 1 oder 2, dadurch gekennzeichnet, daß das Aluminium Eisen in einer Menge von etwa 0,02 bis etwa 0,1 Gew.-% enthält.

**5.** Verfahren nach einem der vorangehenden Ansprüche, dadurch gekennzeichnet, daß das Aluminium im Überschuß bezogen auf die auf eingesetzte Säure berechnete stöchiometrische Menge vorliegt.

**6.** Verfahren nach einem der vorangehenden Ansprüche, dadurch gekennzeichnet, daß das Aluminiuhhalogenidhydrat durch Sprühtrocknung der heißen Lösung gewonnen wird.

**7.** Verfahren nach einem der vorangehenden Ansprüche, dadurch gekennzeichnet, daß die Konzentration des Produkts in der Lösung etwa 8 bis etwa 15 Gew.-% beträgt, die Reaktionstemperatur auf etwa 75 °C bis etwa 85 % gehalten wird und die Reaktionszeit unter 24 h beträgt.

**8.** Verfahren zur Herstellung eines Aluminium-Zirkonium-Hydroxyhalogenidglucinat-Komplexes mit der empirischen Formel:

$(Al_2(OH)_{6-y}X_y)_a(ZrO(OH)_xCl_{2-x})_b$ Neutrale Aminosäure,

in der x einen Zahlenwert von 0 bis 1,5, Y einen Zahlenwert von 0,7 bis 3,0, X Chlor, Brom oder Jod bedeutet und a und b so ausgewählt werden, daß das Al/Zr-Atomverhältnis zwischen etwa 2,0 bis etwa 8,0 beträgt, dadurch gekennzeichnet, daß es folgende Schritte umfaßt:

(a) Einsatz einer wäßrigen Lösung von Komponente A, umfassend 8 bis 35 % eines basischen Aluminiumhalogenids, bei einer Temperatur von 45 bis 100 °C, welche die empirische Formel aufweist,

$Al_2(OH)_{6-y}X_y \bullet nH_2O$

in der y einen Zahlenwert von etwa 0,7 bis etwa 3, X Chlor, Brom oder Jod, n einen Zahlenwert von etwa 0,8 bis etwa 4 bedeutet, und dessen Polymerverteilung, wie charakterisiert durch einen Größenexklusionschromatographie-Test, mindestens 25% seiner Polymere in Bande III und 100 % der aluminiumhaltigen Polymere in den Banden II, III und IV aufweist;

(b) Einsatz einer wäßrigen Lösung der Komponente B, umfassend eine 20 bis 55%ige Lösung von Zirconylhydroxychlorid der empirischen Formel:

$ZrO(OH)_xCl_{2-x}$

in der x einen Zahlenwert von 0 bis 1,5 besitzt;

wobei eine neutrale Aminosäure in einer der beiden Lösungen der Komponenten A oder B oder in beiden Lösungen enthalten ist;

(c) Mischen der Komponenten A und B bei einer Temperatur von 50 bis 100 °C für eine Zeit von weniger als 2 h zur Herstellung einer vereinigten Lösung, wobei ein Komplex von Komponente A und Komponente B und Aminosäure gebildet wird, und

(d) Sprühtrocknung des Komplexes zu einem Pulver mit einer Polymerverteilung, wie gekennzeichnet durch den Größenexklusionschromatographie-Test, von

1) einem Peak-Höhenverhältnis von Peak 4 zu Peak 3 von 0,5 bis 1,8:1 und

2) Peaks (1 + 2) von weniger als 4 Gew.-% der Polymerverteilung.

**9.** Verfahren nach Anspruch 8, dadurch gekennzeichnet, daß der Mischschritt (c) weiter die Zugabe einer neutralen Aminosäure zu Komponente (A) in einer derartigen Menge umfaßt, daß das Atomverhältnis von Aminosäure zu Aluminium etwa 0 bis etwa 3,0:1 beträgt.

**10.** Verfahren nach Anspruch 8, dadurch gekennzeichnet, daß der Mischschritt (c) weiter die Zugabe einer neutralen Aminosäure zu Komponente (B) in einer derartigen Menge umfaßt, daß das Atomverhältnis von Aminosäure zu Zirkonium etwa 0 bis 3,0:1 beträgt.

**11.** Verfahren nach einem der Ansprüche 8 bis 10, dadurch gekennzeichnet, daß die Temperatur vor der Mischung der Komponente A etwa 85 bis etwa 98 °C und die Temperatur der Komponente B etwa 30 bis etwa 50 °C beträgt.

**Revendications**

**1.** Procédé pour préparer un halogénure basique d'aluminium ayant la formule brute ou empirique :

$Al_2(OH)_{6-y}X_y pnH_2O$

(dans laquelle n a une valeur numérique d'environ 0,8 à environ 4, X représente le chlore, le brome ou l'iode; et y a une valeur numérique d'environ 0,7 à environ 3), procédé caractérisé en ce qu'il comprend :

(a) la réaction de l'aluminium métallique avec un hydracide halogéné de formule HX (dans laquelle X représente le chlore, le brome ou l'iode) à une température d'environ 50 °C a environ 100 °C durant environ 1 à environ 100 heures, la concentration du produit dans la solution étant d'environ 8 % à environ 35 % en poids de la solution; et

(b) la récupération du composé basique d'aluminium à partir de la solution chaude, la distribution des polymères du produit formé, telle que caractérisée par une chromatographie avec

17

exclusion en fonction de la taille, étant :

(c) 100 % des polymères se trouvent dans les bandes II, III et IV, et l'on ne trouve sensiblement pas de produit dans la bande I et,

(d) la bande III contient au moins 25 % du polymère.

2. Procédé selon la revendication 1, caractérisé en ce que l'aluminium est sous forme de pastilles ou de poudre.

3. Procédé selon la revendication 1 ou la revendication 2, caractérisé en ce que l'aluminium contient du cuivre en une quantité d'environ 0,005 à environ 0,03 % en poids.

4. Procédé selon la revendication 1 ou la revendication 2, caractérisé en ce que l'aluminium contient du fer en une quantité d'environ 0,02 à environ 0,1 % en poids.

5. Procédé selon l'une quelconque des revendications précédentes, caractérisé en ce que l'aluminium est présent en excès de la quantité stoéchiométrique, calculée sur la base de l'acide que l'on utilise.

6. Procédé selon l'une quelconque des revendications précédentes, caractérisé en ce que l'halogénhydrate d'aluminium est récupéré par séchage par atomisation de la solution chaude.

7. Procédé selon l'une quelconque des revendications précédentes, caractérisé en ce que la concentration du produit dans la solution vaut environ 8 à environ 15 % en poids, la température du mélange réactionnel est maintenue à environ 75°C à environ 85°C, et le temps de réaction est inférieur à 24 heures.

8. Procédé pour préparer un complexe hydroxyhalogénure de zirconium/glycinate d'aluminium, ayant la formule brute ou empirique :

$(Al_2(OH)_{6-y}X_y)_a(ZrO(OH)_xCl_{2-x})_b$ Amino acide neutre

(dans laquelle x a une valeur numérique de 0 à 1,5; y a une valeur numérique de 0,7 à 3,0; X représente le chlore, le brome ou l'iode; et a et b sont choisis de façon que le rapport atomique Al/Zr se situe entre environ 2,0 et environ 8,0, procédé caractérisé en ce qu'il comprend :

(a) l'introduction d'une solution aqueuse d'un constituant A, comprenant 8 à 35 % d'un halogénure basique d'aluminium, à une température de 45 à 100°C et ayant la formule brute ou empirique :

$Al_2(OH)_{6-y}X_y.nH_2O$

(dans laquelle y a une valeur numérique d'environ 0,7 à environ 3; X représente le chlore, le brome, ou l'iode; et n a une valeur numérique d'environ 0,8 à environ 4) et dont la distribution des polymères, telle que caractérisée par un essai de chromatographie avec exclusion en fonction de la taille de molécule, comporte au moins 25 % de ses polymères en bande III et 100 % des polymères contenant l'aluminium présents dans les bandes II, III et IV;

(b) l'introduction d'une solution aqueuse du constituant B, comprenant une solution à 20-55 % d'hydroxychlorure de zirconyle ayant la formule brute ou empirique :

$ZrO(OH)_xCl_{2-x}$

(dans laquelle x a une valeur numérique de 0 à 1,5), un amino acide neutre étant inclus dans l'une des deux solutions, ou dans les deux solutions, des constituants A et B;

(c) le mélangeage du constituant A et du constituant B à une température de 50°C à 100°C pendant une période de temps inférieure à deux heures pour former une solution combinée, en formant ainsi un complexe du constituant A et du constituant B et de l'amino acide; et

(d) le séchage par atomisation du complexe, ce qui donne une poudre ayant une distribution des polymères, telle que caractérisée par un essai de chromatographie avec exclusion en fonction de la taille des molécules, présentant:

(1) un rapport entre la hauteur du pic 4 et celle du pic 3 de 0,5 à 1,8:1; et

(2) des pics (1 + 2) représentant moins de 4 % de la distribution pondérale des polymères.

18

9. Procédé selon la revendication 8, caractérisé en ce que ladite étape de mélangeage (c) comprend en outre l'addition d'un amino acide neutre au constituant A, addition effectuée en une quantité telle que le rapport atomique de l'amino acide à l'aluminium se situe entre environ 0 et environ 3,02:1.

10. Procédé selon la revendication 8, caractérisé en ce que ladite étape de mélangeage (c) comprend en outre l'addition d'un amino acide neutre au constituant B, addition effectuée en une quantité telle que le rapport atomique de l'amino acide au zirconium se situe entre environ 0 et environ 3,02:1.

11. Procédé selon l'une quelconque des revendications 8 à 10, caractérisé en ce que ladite température est, avant le mélangeage du constituant A, d'environ 85 à 98°C environ, et la température du constituant B est d'environ 30 à environ 50°C.

# FIG.1.

## CONVENTIONAL 50% ACH

POLYMER DEVELOPMENT AT
VARIOUS PRODUCT STAGES
USING THE INVENTION PROCESS

**FIG.2.**

Aluminum Dichlorhyd-
rate Stage

At 0.85:1 Al/Cl
Atomic Ratio

**FIG.3.**

Aluminum Sesquichlor-
hydrate Stage

At 1.8:Al/Cl Atomic
Ratio

**FIG.4.**

Aluminum Chlorhyd-
rate Stage

At 1.94:1 Al/Cl
Atomic Ratio

Band IV

FIG.5A.

Band IV

Band II

FIG.5B.

Band II

Band IV

FIG.5C.

FIG.6A.

FIG.6B.

FIG.6C.

FIG.6D.

CHROMATOGRAMS ON ALUMINUM/ZIRCONIUM HYDROXYCHLORIDE
GLYCINATE SALTS CONVENTIONAL ALUMINUM/ZIRCONIUM-
GLYCINATE SALTS

FIG.7.

FIG.8.

Ratio Height Peak 4/Height Peak 3: < 0.2: 1
Percent Polymers in Peaks (1÷2): 16.71%

Ratio Height Peak 4/Height Peak 3: 0.2: 1
Percent Polymers in Peaks (1÷2) 27.19%

EP 0 393 275 B1

FIG. 9.

Ratio Height Peak 4/Peak 3: 1.0:1
Percent Polymers in Peaks (1+2): 0.43%

FIG.10.

Ratio Height Peak 4/Height Peak 3: 1.0:1
Percent Polymers in Peaks (1÷2): 0.57%

GILLETTE'S IMPROVED ALUMINUM/ZIRCONIUM GLYCINATE SALT
VIA U.K. PATENT NO. 214499.2A

FIG.11.

FIG.12.

I Hour at 95°C
Ratio Height Peak 4/Height Peak 3: 3.9:1
Percent Polymers in Peaks (I+2): I0.98%

4 Hours at 95°C
Ratio Height Peak 4/Height Peak 3: 2.9:1
Percent Polymers in Peaks(I+2): I6.03%

EP 0 393 275 B1